# EUROPEAN PATENT APPLICATION

(11) **EP 4 541 340 A1**
(43) Date of publication of application: **23.04.2025**
(21) Application number: 24206799.9
(22) Date of filing: 15.10.2024
(51) Int. Cl.: A61K 8/02, A61K 8/30, A61K 8/31, A61K 8/34, A61K 8/37, A61K 8/44, A61K 8/46, A61K 8/92, A61Q 19/10

(54) **A ONE POT PROCESS FOR PREPARING 'GRIT-FREE' SOLID CLEANSING COMPOSITIONS**

(30) Priority: 21.10.2023 IN 202321072052
(71) Applicant: Galaxy Surfactants Ltd., Navi Mumbai 400 703 Maharashtra (IN)
(72) Inventor: KOSHTI, Nirmal, New Jersey, 08854 (US); ABHICHANDANI, Bharat Ghanshyamdas, 413801 Daund (Pune), Maharashtra (IN); SAWANT, Bhagyesh Jagannath, 421306 Katemanvali, Kalyan (E), Maharashtra (IN); SHINDE, Nitesh Suresh, 400701 Navi Mumbai, Maharashtra (IN); JOSHI, Pranali, 400705 Navi Mumbai, Maharashtra (IN); GOSAVI, Kiran Prakash, 421201 Dombivli East, Maharashtra (IN); SHIRKE, Prashant Prabhakar, 421201 Dombivli East, Maharashtra (IN); TALUKDAR, Sukanya, 400615 Waghbil Naka, Thane (W), Maharashtra (IN); SAVLA, Parag Narendra, 421302 Bhiwandi, Maharashtra (IN)
(74) Representative: Müller Schupfner & Partner Patent- und Rechtsanwaltspartnerschaft mbB (Hamburg)

(57) **Abstract**

The invention relates to a one pot process for preparing a 'grit-free' solid cleansing composition. The composition has pH resembling to skin pH which comprises hydrogenated triglyceride oil/s, humectant, sodium *O*-acyl isethionate as anionic surfactant, polyglyceryl ester, *N*-acyl glycine/s, water and, skin benefit agents and/or hair benefit agents. This is accomplished by a unique process using sodium *O*-acyl isethionates of particle size less than 250 µm.

## Description

### FIELD OF INVENTION:

The present invention relates to a one pot process of preparing a 'grit-free' solid cleansing composition. More particularly, the present invention relates to a one pot process of making solid cleansing bars with high vegetable oil (emollient) content. The one pot process of the present invention affords 'grit-free' solid cleansing bars by extruding a base that is conveniently made by blending hydrogenated vegetable oil, glycerine with sodium O-acyl isethionates, polyglyceryl ester, water and *N*-acyl glycines.

### BACKGROUND OF THE INVENTION:

Conventional soaps bar cakes (sodium or potassium salts of fatty acids) are highly alkaline in nature. The pH of human skin ranges from 5 to 6, and hence the soaps with highly alkaline pH (10 to 11) temporarily change the skin pH which affects the friendly skin microbiota. Also, the harsh nature of soap disturbs the integrity of the stratum corneum (and hence the barrier function) by interacting with constituents (proteins, lipids and NMF (natural moisturising factor)) of the stratum corneum leading to dryness of skin. The problems arising out of high alkalinity of typical soaps are addressed to some extent by synthetic surfactants (like sodium lauroyl and sodium coco sulphate) sulphate which is available in dry form for solid compositions. However, sulphates surfactants with very strong anionic charge are known for denaturing and dissolving proteins. The 'sulphate-type' anionic surfactants in general compromise the integrity of skin's uppermost layer, the stratum corneum. This leads to dryness (increased rate of water loss due to weakened stratum corneum) and irritation of the skin. Sulphate-surfactants top the irritancy index and hence the strong attempt/trend in the last decade by the industry was to create cleansers for personal care that are `sulphate-free'. This problem due to harshness of soap and 'sulphate-surfactants' was addressed by 'syndet' bars where part of the conventional soap (sodium fatty acid carboxylate) was replaced by sodium cocoyl isethionate. US patent 2,894,912 (1959) describes `Isethionate detergent bar' which predominantly has sodium cocoyl isethionate in it, and free fatty acid as binder/plasticizer in addition to a small percentage of conventional soap and water. Though the personal cleansers with harsh surfactants like soaps and `sulphates' have not vanished totally (these are relatively inexpensive compared to other `sulphate-free' surfactants), the last decade has seen several cleansers for both hair and skin care that are without `sulphate-surfactants' and 'soap'. Universally, the major surfactant of these `sulphate-free and soap-free' cleansers is sodium *O*-acyl isethionate.

The major problem of the sodium *O*-acyl isethionate surfactant is the difficulty in formulating the same due to its hard nature and its poor solubility in water. Acyl isethionates are readily commercially available in flakes and noodle/needle form with about 80 to 85 % active matter, the remainder being unreacted sodium isethionate and the fatty acid. Formulating with sodium cocoyl isethionate, both in liquid and solid formats, necessitates mixing of ingredients at elevated temperatures of 70 to 90°C in the presence of water. In case solid cleansers like bars, the problem gets worse since the unsoftened particles show up during bathing / cleansing operation on the surface as 'grits'. These tiny but tough particles not only spoil the aesthetic value by appearing on the surface of the solid products but sometimes, they can cause serious harm to skin (can lacerate the skin during use of soap on body, small but hard speck can scratch the skin while applying soap on the body) during skin cleansing activity. The problem of eliminating grit (avoiding its formation during the processing) from the cleansing bars becomes acute since this form of personal cleansers typically employ very limited quantity of water as a part of the formulation. By the same token, the solid formulations with high hydrophobic (oily) ingredients pose the increased difficulty in avoiding formation of grit. Under these circumstances, a lesser quantity (lower percentage) of water and higher quantity (higher percentage) of hydrophobic material complement each other in hindering the softening process of inherently tough sodium O-acyl isethionate. This problem is discussed in GB 1294754 that teaches the incorporation of emollient oils shouldn't exceed 15 % by weight of the total composition since it results in the grittiness in the final bar composition as well as the depression of lather. It is also reported that the higher % of the emollient oils (above 15 %) can result in 'cracking' of the bar cakes.

The grit in the cleansing tablets mainly results from hard nature of sodium cocoyl isethionate which doesn't soften enough (less hydration of sodium *O*-acyl isethionate due less water available, and also due to a lot of hydrophobic emollient) in the `conventional processing' .

This problem has been addressed in US Patent 11,446,216 which teaches creating of a uniform emulsion comprising the 1) hydrophobic saturated triglyceride oils, 2) sodium *O-*cocoyl isethionate, 3) a non-ionic emulsifier and 4) about 30% of water. The salient features of this emulsification process are,
1) use of non-ionic emulsifiers of HLB value between 8 to 9
2) use of high amount of water during emulsification and subsequent removal of part of the originally added quantity while it is being cooled and solidified, and
3) high temperature of emulsification

The method entails preparing an emulsion of the above mentioned four components at elevated temperature (90°C) for several hours using excess water (25-30% by weight of the total composition taken for emulsification) and subjecting the emulsion to cooling and solidification during which the water content is reduced to 10% or less by weight of the total solid composition as against 30-35% by weight of the total composition in the beginning of emulsification process). The typical process involves heating of sodium cocoyl isethionate, a hydrogenated vegetable oil, a non-ionic emulsifier and higher amount of water together at 90°C for five hours and the hot emulsion is then passed through a needle plate with 0.5 mm mesh size. Almost 25% of original water content is lost in the evaporative cooling that is accompanied by solidification of the material. The solid material thus obtained, is amenable to plodding /extrusion to convert it into billets that can be converted to tablets/bars by stamping.

The high temperature and the longer duration of emulsification with large quantity of water result in hydrolysis of sodium cocoyl isethionate which is an ester as shown below. Sodium cocoyl isethionate, just like any other ester, is highly susceptible to hydrolysis and rate of hydrolysis is dependent on all the three factors, namely, the water content, the temperature and the time duration. Slight acidity or alkalinity accelerates (catalysis) the hydrolysis (Scheme 1). This strong possibility of acid catalyzed hydrolysis of esters in the presence of large quantity of water and higher temperatures also narrows the scope of manufacture of solid cleansers where acidic pH with incorporation of some of the actives that are acidic in nature. Acidic pH of certain compositions is essential requirement for the best efficacy, for example, feminine intimate hygiene (pH 3.0 to 4.5) or anti-acne (pH 3.5 to 4.5) cleansers or even regular hair and skin cleansers with pH of 5.5 which is average pH of human skin.

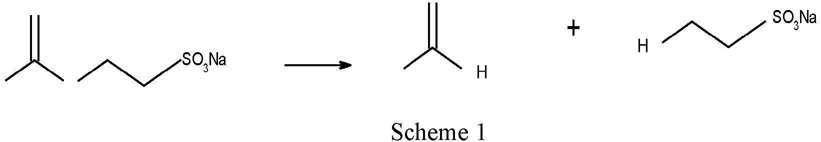

In summary, the process of US Patent 11,446,216 employs the solid form of sodium cocoyl isethionate to begin with and converts it into the final 'non-gritty' form via a complex emulsification (liquid) process to again convert into solid form by specialized equipment where the excess water needs to be evaporated. This has 'cost-implication' for the final output due both the capital expenditure as well as rate of throughput in addition to loss of `active' matter due to hydrolysis of an extremely susceptible `ester' type surfactant.

There is a definite need to design an efficient yet simple process to get 'grit-free' solid cleanser with high hydrophobic ingredients without resorting to an elaborate emulsification with adding excess water and then removing it using a 'special' equipment for evaporative cooling of the mass.

This problem of 'grits' originating from sodium cocoyl isethionate is addressed by Koshti et al. (IN202321055642) by synthesizing sodium cocoyl isethionate in the presence of sodium cocoyl methyl isethionate using cocoyl chloride and dry powders of sodium isethionate and sodium methyl isethionate at relatively low temperature (65°C). This kind of co-synthesis of sodium cocoyl isethionate with other surfactants like sodium cocoyl methyl isethionate or sodium cocoyl methyl taurate from fatty acids at high temperature of around 200 °C, is possible but the process by Koshti *et al.* avoids formation of high temperature induced degraded products.

Hence there is a need to develop a process for preparing grit free solid cleansing composition which requires lower water content, lower processing time and that will be easy to manufacture at the same time.

The inventors of the present invention, unexpectedly discovered that simply using of sodium cocoyl isethionate in `fine powder' form affords 'high emollient oil' containing solid cleansers that are gritless without resorting to elaborate emulsification process of the prior art. The inventors of present invention discovered the incorporation of skin-hair hygiene agents, N-acyl glycines, with the desired pH range of 4 to 5.5 in these 'grit-free' solid skin-hair cleansing compositions.

### OBJECTIVES OF THE INVENTION:

It is an objective of the present invention to overcome the limitations of the prior art for making 'non-soap' based 'grit-free' solid cleansing compositions with high incorporation of vegetable oils.

Another objective of the present invention is to create a one pot process for `grit-free' and 'high oil content' solid cleansing systems using sodium O-acyl isethionate without resorting to high temperature emulsification process and subsequent concentrating the emulsion by evaporative cooling using a special equipment.

Another objective of the present invention is to create a one pot process that would eliminate the possibility of loss of `active matter' due to hydrolysis while achieving final composition which is totally free from grits. This necessitates an approach that would avoid heating of `ester' type *O*-acyl isethionate with water at higher temperature.

Yet another objective of the present invention is to create `soap-free' cleansing compositions for skin and scalp hygiene with pH similar to skin pH to prevent the perturbation of skin microbiota.

Yet another objective of the present invention is to create `soap-free' cleansing compositions for anti-body odor, and anti-dandruff effect.

Yet another objective of the present invention is to provide a one pot process that would allow to produce solid cleansers with acidic pH for applications like anti-acne and feminine intimate hygiene washes where the ideal pH range is very acidic ranging from 3.0 to 4.0.

### SUMMARY OF THE INVENTION:

In an aspect the present invention provides a grit-free solid cleansing composition comprising:
a) at least 30 %, by weight of the total composition, one or more hydrogenated vegetable oil;
b) at least 4% by weight of the total composition, a humectant;
c) at least 30 %, by weight of the total composition, a salt of *O*-acyl isethionate;
d) at least 5 %, by weight of the total composition, a polyglyceryl ester of fatty acid;
e) at least 0.4 % by weight of the total composition, one or more N-acyl glycine; and
f) less than 5 % by weight of the total composition, water,

wherein, pH of the grit-free solid cleansing composition at 5 % dispersion in water,
is between 3.5 to 6.0,
wherein melting point of the hydrogenated vegetable oil is at least 45 °C; and wherein the salt of O-acyl isethionate is in powder form with particle size less than 250 µm.

In another aspect the present invention provides a grit-free solid cleansing composition comprising:
a) 30 % to 40 % by weight of the total composition, one or more hydrogenated vegetable oil;
b) 4% to 10 % by weight of the total composition, a humectant;
c) 30 % to 45 % by weight of the total composition, a salt of *O*-acyl isethionate;
d) 5 % to 12 % by weight of the total composition, a polyglyceryl ester of fatty acid;
e) 0.4 % to 4 % by weight of the total composition, one or more *N*-acyl glycine; and
f) less than 5 % by weight of the total composition, water,

wherein, pH of the grit-free solid cleansing composition at 5 % dispersion in water,
is between 3.5 to 6.0,
wherein melting point of the hydrogenated vegetable oil is at least 45 °C; and wherein the salt of O-acyl isethionate is in powder form with particle size less than 250 µm.

In yet another aspect the present invention provides a one pot process for preparing grit-free solid cleansing composition comprising:
a) at least 30 %, by weight of the total composition, one or more hydrogenated vegetable oil;
b) at least 4% by weight of the total composition, a humectant;
c) at least 30 %, by weight of the total composition, a salt of *O*-acyl isethionate;
d) at least 5 %, by weight of the total composition, a polyglyceryl ester of fatty acid;
e) at least 0.4 % by weight of the total composition, one or more *N*-acyl glycine; and
f) less than 5 % by weight of the total composition, water,

wherein, pH of the solid composition at 5 % dispersion in water, is between 3.5 to 6.0,
wherein melting point of the hydrogenated vegetable oil is at least 45 °C;
wherein the salt of O-acyl isethionate is in powder form with particle size less than 250 µm; and
wherein the process comprises the steps of:
   A. mixing component a) and b) at temperature between 90 to 95 °C, to afford a homogenous mass;
   B. adding component c) to the homogenous mass of step A to obtain a reaction mass;
   C. cooling the reaction mass of step B and adding components d) to f) followed by kneading to afford an amalgamated mass;
   D. processing the amalgamated mass of step C into a billets; and.
   E. processing the billets obtained in step D into the solid cleansing composition.

The above-described features and the advantages of the present disclosures will be appreciated and understood by those skilled in the art from the `detailed description' and the `claims'.

### DETAILED DESCRIPTION OF THE INVENTION:

The term `soap-free' used herein refers to the composition which does not have any soap component. Soap includes all the salts of a fatty acid or triglyceride oils that are known to person skilled in art. Few representative examples are sodium laurate, potassium laurate, sodium palmate, and sodium cocoate.

The term `sulphate free' as used herein refers to composition which is devoid of sulphate-based surfactants that are commonly known in the art. Commonly known sulphate-based surfactants include sodium lauroyl sulphate, sodium coco sulphate, ammonium lauroyl sulphate, etc.

The term 'grit-free' as used herein refers to composition which is devoid of any unsoftened particles of sodium *O*-acyl isethionates that are commonly referred to as 'grits'. Grits are tiny particles that provide an unpleasant sandy feel during use of solid cleansing compositions.

The symbol µ or µm, can be used interchangeably and as used herein in the entire specifications refer to the particle size in micrometre.

Manufacturing of 'high oil' containing solid form (bar cakes/tablets) of personal cleansing composition by emulsifying the oily components (that are solids at or below 45°C) with water using non-ionic surfactants with HLB value of 6 to 12 is known in the art (US Patent 11446216).

The 'water-in-oil' type emulsions, described in the prior art, are made at temperature of 90°C using very high % of water. Typically, the initial quantity of water taken for emulsification is around 25 % by weight of the total composition which is made up of 'soap-free' surfactant, hydrogenated oil, non-ionic surfactant along with water. This high level of water and the high temperature are needed for proper emulsification and softening of anionic sodium *O*-acyl isethionate surfactant. The emulsion, thus obtained, is then cooled and solidified with concurrent loss of water to ensure that the final solid composition has minimum 7% water. The hot flowable emulsion is pumped through a needle plate with very fine holes of 0.5 mm mesh. During this process almost 30 to 60% of original water is lost by evaporation resulting in the final content of water to be around 10% of by weight. Emulsifiers taught in the prior art are polyglyceryl-4 palm kernelate, glyceryl monostearate, sorbitan monooleate, and polysorbate 20. The prior art teaches inclusion of at least 3 % of one emulsifier or a combination of emulsifiers resulting in the desired HLB value of 6 to 12 that are based on polyglyceryl esters of fatty acids or ethoxylated sorbitan esters of fatty acids. The triglyceride oil phase has been 16 to 35 % of the final composition.

Thus, the process of the prior art involves emulsification of water and oil with non-ionic emulsifiers of a particular HLB value. This process is carried out at high temperatures of 90°C for long hours after the sodium cocoyl isethionate softens. Commercially available sodium cocoyl isethionate (or sodium lauroyl isethionate) is in the form of needles or flakes and its melting point is around 190-200°C. Hence it is important that every particle of sodium *O*-acyl isethionate is softened, otherwise the resultant solid cleanser carries unsoftened particles of sodium *O*-acyl isethionates that are commonly referred to as 'grits'. The process described in the prior art, although lengthy and cumbersome, ensures 'grit-free' solid cleansers. After softening of sodium salts of *O*-acyl isethionate and emulsification using high temperatures and plenty of water, and the water needs to be removed (30 to 40 % of the original quantity) during the process of cooling the mass and converting it into solid form using a special equipment.

The inventors of the present invention have developed a one pot process of preparing 'grit-free', `sulphate-free' and 'soap-free' solid cleaners without resorting to creating dilute emulsion with excess amount of water first and then subsequent concentrating it (evaporation of excess water) before the solid mass is made suitable for its conversion into solid cleansers.

The one pot process of the present invention involves blending of hydrogenated triglyceride oils with humectant, salt of O-acyl isethionate in the fine powder form with particle size of less than 250 µm (100% particles to be < 250 µm), polyglyceryl ester of fatty acid, *N*-acyl glycines and water. The one pot process of the present invention not only obviates the step of emulsification involving significant percentage of water, but it also rules out any possibility of the loss of activity due to large amount of water (being present during emulsification) and the temperature induced hydrolysis of anionic surfactant which is an ester type and hence highly susceptible to hydrolysis at high temperatures of 90 °C.

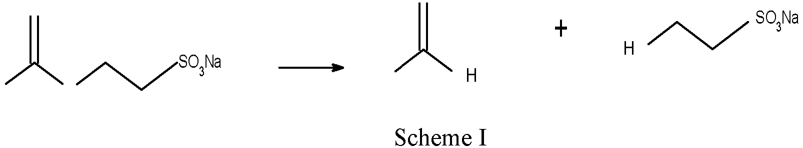

The above blended mass produced by the one pot process disclosed in the present invention is amenable to conventional soap-manufacturing process (kneading/refining, triple roll milling/plodding) to deliver 'grit-free' bar cakes/tablets or any other solid form with pH like skin pH or even lower pH required for special wash applications.

Suitably the one pot process for preparing grit free solid cleansing composition of present invention comprises following steps:
A. mixing component a) and b) to afford a homogenous mass;
B. adding component c) to the homogenous mass of step A to obtain a reaction mass;
C. cooling the reaction mass of step B and adding components d) to f) followed by kneading to afford an amalgamated mass;
D. processing the amalgamated mass of step C into a billets; and.
E. processing the billets obtained in step D into the solid cleansing composition.

Suitably the step A of the one pot process of present invention involves the mixing of components a) and b) of the solid cleansing composition.

Suitably the step A is carried out at temperature of about 90 to 95 °C. Suitably when component a) and b) are mixed, the mixture so obtained is a homogenous mixture. Suitably step A may require the stirring to obtain a homogenous mixture. Suitably the Step A is carried out in a high shear mixer blender. Suitably the high shear mixer blend is sigma mixture comprising sigma-shaped blades.

Suitably the component d) to component f) are absent in the step A.

Suitably step B of the one pot process of present invention involves adding of component c) to the homogenous mass of step A. Suitably the stirring is continued for about 3 hours.

Suitably the component c) is added in the form of fine powder form. Suitably the fine powder of the component c) is of particle size less than 250 µm. Suitably all the particles of component c) have the particle size of less than 250 µm. Suitable 100 % of the particles of component c) have particle size distribution to be < 250 µm. In a preferred embodiment the particle size is as low as 1 µm.

Suitably step B is carried out at temperature of about 90 to 95 °C.

Suitably step B is devoid of component d) and component f).

Suitably the step B affords a homogenous reaction mass.

Suitably the step C of the one pot process of present invention involves cooling the homogeneous reaction mass to about 60 to 65 °C. Suitably the reaction mass of step C is under continuous kneading in the sigma mixture. Suitable consistency of mass so obtained after cooling is semisolid paste. Suitable the consistency of mass obtained after cooling is in the form of dough. The component d) to component f) are added in step C during the continuous kneading of the reaction mass. Suitably the reaction mass after addition of component d) to component f) is further kneaded for about 4 hours to afford an amalgamated mass. Suitably the temperature during the step C of the one pot process of present invention is maintained at 65 °C. Suitably the temperature during the step C is not exceeded above 65 °C. Suitably the additive and active can be added in the solid cleansing composition of the present invention at step C of the one pot process of the present invention.

Suitably the amalgamated mass of step C is cooled to 30 °C during the step D of the one pot process of present invention. Suitably step D involves constant kneading in the sigma mixture. Suitably the kneaded mass is processed using a triple roll mill and duplex plodder. Suitably the mass, after processing in triple roll mill and a plodder, is extruded into billets. Suitably the billets obtained in the one pot process step D is further processed in the step E into the solid cleansing composition. Suitably during the step E of the one pot process of present invention the billets are stamped using a conventional stamping machine, used for soap manufacturing, into a solid cleansing composition. Suitably solid cleansing composition obtained after stamping is a solid cleansing bar or solid cleansing tablet.

The one pot process of the present invention gives an ease in manufacturing of grit free solid cleansing composition using salt of O-acyl isethionate preferably sodium cocoyl isethionate and sodium lauroyl isethionate. Furthermore, the one pot process also requires less amount of water and eliminates the need of higher processing time as required by prior art.

### Hydrogenated vegetable oil:

As used herein the hydrogenated vegetable oil, component a) of the grit free solid cleansing composition of present invention, is selected from any hydrogenated vegetable oil that has melting point of 45 °C or higher. The melting point can be in the range 45-90°C.

Preferably the hydrogenated vegetable oil is used alone or is used in combination of another hydrogenated vegetable oil. Preferably the one or more hydrogenated oil is selected from hydrogenated sunflower oil, hydrogenated soyabean oil, hydrogenated palm-stearin, hydrogenated cotton seed oil, hydrogenated rapeseed oil, hydrogenated olive oil, hydrogenated coconut oil, hydrogenated palm kernel oil or mixture thereof. Example 6 uses hydrogenated sunflower oil whereas Examples 8, 9, 10,11 and 13-17 use hydrogenated palm-stearin. The rest of the examples are with hydrogenated soyabean seed oil. Hydrogenated soyabean oil, hydrogenated sunflower seed oil, hydrogenated palm stearin and hydrogenated cotton seed oil are commercially readily available, however, it is very clear to a skilled person in the art that any hydrogenated vegetable oil or the blends thereof, can be used in the one pot process of the present invention provided the criterion of melting point of 45°C or above is met with.

Hydrogenated vegetable oils of the present invention can be one or it can be a combination of more than one hydrogenated triglyceride oils. Solid cleanser made with hydrogenated palm-stearin (melting point 60°C) seems to give advantage with hardness and less mush (Example 8, table of analysis). Rapeseed oil, olive oil, coconut oil, palm kernel oils are widely produced, and their hydrogenated versions are commercially available.

The fully saturated forms of these vegetable oils are selected for the solid cleanser compositions of the present invention. Palm stearin is a fraction of palm oil is obtained from crystallization of palm oil. The crystallized fractions contain high percentage of saturated fatty acids and relatively low percentage of unsaturated fatty acids. This is further hydrogenated to get saturated C₁₆ and C₁₈ based triglycerides. Hydrogenated palm-stearin used in Examples 8,9,10,11 & 13-17, has melting point of 60°C and iodine value of less than one. Oils with higher % of long fatty acids (above C₁₈ to C₂₂) have higher melting point of up to 80°C, and they are suitable for solid cleansing forms of the present invention.

Suitably the hydrogenated vegetable oil is present in an amount of at least 30 % by weight of the solid cleansing composition.

Suitably the hydrogenated vegetable oil is present in an amount of at least 30 % and up to 40 % by weight of the solid cleansing composition.

### The Humectant:

As used herein 'humectant', component b) of the solid cleansing composition of present invention are preferably selected from glycerin, sorbitol, polyglycerin, or any other natural liquid molecules with multiple hydroxyl functionality. Plant derived glycerin is the polyglyceryl ester used in the first 'non-aqueous' step, step A, of the one pot process of the present invention. Glycerin is a proven skin moisturizer in personal care compositions. Glycerin polymers (or polyglycerin) are commercially available and not only they provide good humectancy but also provides a good binding effect for solid cleansing composition of the present invention. Polyglycerin-3 (CAS No 56090-54-1) and polyglycerin-4 (CAS No 56491-53-3) replace glycerin (that is present in Examples 1-13) in Example 14 and Example 15 respectively. Sustainable diols, or triols or small molecules with several hydroxyl groups including triose, tetraose, pentose or hexose can be used. Fermentation derived 1,3 propane diol (CAS no 504-63-2) (from glycerin by fermentation route using *Lactobacillus reuteri)* can be selected for the role of humectant (Example 16). These molecules and the water molecules bound to them can help maintain the integrity of the final form of the composition.

Suitably humectants are present in an amount of at least 4% by weight of the solid cleansing composition.

Suitably humectants are present in an amount of at least 4% and up to 10 % by weight of the solid cleansing composition.

### Salt of O-acyl isethionate:

As used herein "salt of *O*-acyl isethionate", component c) of the solid cleansing composition of present invention, refers to an anionic surfactant molecule. The examples of salts of *O*-acyl isethionate includes alkali metal salt O-acyl isethionate. Preferably alkali metal salts of *O*-acyl isethionate are selected from sodium cocoyl isethionate, sodium lauroyl isethionate, potassium cocoyl isethionate, potassium lauroyl isethionate or mixture thereof. Most preferable alkali metal salts of *O*-acyl isethionate are sodium cocoyl isethionate and sodium lauroyl isethionate.

In an embodiment, the solid cleansing composition of present invention comprises at least 30 weight percent of alkali metal salts of *O*-acyl isethionate. Preferably, the composition comprises about 30 to about 50 weight percentage of alkali metal salts of *O*-acyl isethionate.

Preferably, the solid composition of present invention employs alkali metal salts of *O*-acyl isethionate in powder form. In an embodiment the particle size of powdered alkali metal salts of *O*-acyl isethionate is below 250 µm. While referring to term below 250 µm means that all (100 %) the particles of alkali metal salts of *O*-acyl isethionate are less than 250 µm in size.

Suitably the component c) is present in an amount of at least 30 % by weight of grit free solid cleansing composition.

Suitably the component c) is present in an amount of at least 30 % and up to 45 % by weight of the solid cleansing composition of present invention.

In an embodiment a combination of sodium *O*-lauroyl isethionate with another 'sulphate-free' surfactant, namely, sodium *N-*lauroyl methyl taurate can be employed as component a) of present invention.

Sodium *O*-acyl isethionates are commercially available with active matter levels of 85 % (Example 1) and 80 % (Example 2, the composition is made with sodium cocoyl isethionate of 80 % anionic active). Examples 5 and 6 illustrate solid cleansers with sodium lauroyl isethionate of 85 % active matter.

A combination of sodium *O*-lauroyl isethionate and another 'sulphate-free' surfactant, namely, sodium *N*-lauroyl methyl taurate (CAS No: 4337-75-1, commercially available as 90 % active, particle size distribution < 250 µm), is illustrated in Example 7. It is obvious to anyone who is familiar with the art that surfactants of any type that are intended to be included in the first non-aqueous blending of molten hydrogenated triglycerides oils and other solid ingredients need to be in the form of fine powder. All anionic surfactants used in the present invention have the particle size < 250 µm, regardless of their alkyl chain distribution or the purity. The examples of such 'soap-free' and `sulphate-free' surfactants are disodium lauroyl sulphosuccinate or any other amino acid-based surfactant. Example 4 is illustrated with hydrogenated soyabean seed oil and sodium cocyl isethionate (85 % active matter). In this example the non-aqueous blending is done at 90-95 °C. Preferably the acyl isethionate surfactant has been softened by heating the powder with hydrogenated vegetable oil and glycerin. However, equally good results are obtained in Example 2 by heating the acyl isethionate powder and the hydrogenated oil along with glycerin at 70-75°C where is sodium cocoyl isethionate of 80 % activity is used with hydrogenated soyabean seed oil. Most experiments have been performed at 90-95°C. When the temperature of the kneaded mass is brought down to 60-65°C, other ingredients like N-acyl glycine, polyglyceryl ester, a chelating agent, an anti-oxidant, any other thermostable ingredient and water are added and mixing is continued. The cooled kneaded mass at ambient temperature is directly taken for refining (triple roll milling) and plodding. Thus, in one pot (Sigma blender) three stages of addition of ingredients at three different temperatures are performed (90-95°C, 60-65°C and 25-30°C) and amalgamated material is then processed on the conventional soap making equipment to either convert it into noodles, or billets to get the bars after stamping.

### Polyglyceryl ester as foam boosters:

As used herein `polyglyceryl ester', component d) of the solid cleansing composition of present invention, are used as foam boosters. Polyglyceryl esters being employed in the present invention are preferably esters of fatty acid. Polyglyceryl esters of fatty acid being employed in the present invention have been used in solid bar cleansers in early 2000's (JP 7-268392 A JP 2002-194389 A ) including transparent bathing bars (JP2005036123).

Use of Polyglyceryl ester in skin cleansers have been reported to reduce the irritancy potential of anionic surfactants (EP 2468246, 2014). Fevola et al. (EP 3181114A1) reported foam boosting properties of polyglyceryl esters, particularly, those with melting points of 40 °C or less in solid cleansing compositions (like bar cakes).

Such polyglyceryl esters with peak melting point less than 40 °C (named as PGE₄₀) are also reported to enhance the mildness (reduced irritation) in addition to boosting of lather, thus making the composition suitable for cleansing of sensitive areas including baby/infant's skin, irritated and compromised skin and feminine intimate cleansing. These are also reported to aid the processibility of bar cake. Preferably, the polyglyceryl esters employed in the present invention are polyglyceryl-4 laurate (CAS No 75798-42-4) and polyglyceryl-3 oleate (CAS No. 33940-98-6). Polyglyceryl-4 laurate is a totally 'plant-derived' syrupy liquid, that offers good emollience to the skin. It is reported to play a role in 'hair-care bar' composition where it is reported to impart conditioning effect and often considered useful in `silicon-free' hair care products (Cosmetic Ingredient Review, cir-info@cir-safety.org). It is a non-ionic emulsifier (HLB 8 to 9) and made from polyglycerin-4 and lauric acid. It is used in 'oil-in-water' type of emulsions (US patent 9409853B2, US10292925B2) as reported in 2014-15. It has been subsequently used as an emulsifier by J. M. Story (US Patent 11,446,216) in the process of creating solid cleansing formulations (bar cakes) with high oil content. Syndets bar cakes with 30-45 % of sodium cocoyl isethionate with hydrophobic binder/emollient around 20-30 % are reported. Usually, the hydrophobic binder is stearyl alcohol, stearic acid or glyceryl esters of stearic acids. Fevola *et al.* teach similar compositions with polyglyceryl esters (EP 3181114A1, 2015) wherein the cleansing bar comprises of sodium cocoyl isethionate (~ 45 %), hydrophobic binder (~35 %), and a polyglyceryl ester (2 to 6 %) and water (~ 8-9 %). EP 3181114 also discloses a variety of hydrophobic binders like hydrogenated vegetable oils in addition to stearyl alcohol and stearic acid. The examples of Fevola's patent application (2015) with polyglyceryl esters (5.0 %) include 45 % of sodium cocoyl isethionate and 35 % of stearic acid (hydrophobic binder) and 9 % of water. Though hydrogenated triglycerides oils are mentioned in EP' 114 as one of the several hydrophobic binders. Subsequently in 2021 Story (US Patent 11,446,216) used hydrogenated vegetable oils with polyglyceryl esters and acyl isethionate at similar percentage level in the solid cleanser composition but with a different way of processing to claim an efficient process via emulsification route.

In many examples of the present invention polyglyceryl-4 laurate is used. Examples 1, 12 and 13 deploy polyglyceryl-3 oleate. Preferably the polyglyceryl esters are added during the step C of the one pot process of present invention. During the step C, the temperature of kneaded mass of hydrogenated oil (component a), glycerin (component b) and anionic acyl isethionate (component c) is around 60 to 65 °C. At this stage there is no classical w/o emulsification intended since water quantity is less than 5.0 % of the total mass and the material in sigma mixer is solid in nature.

Example 4 demonstrates that polyglyceryl esters can be added at the beginning when temperature is 90 °C before the addition of powdered sodium cocoyl isethionate. The addition of polyglyceryl esters at different stages of the process does seem to work well in terms of processibility and in getting gritless bar cakes after stamping. The preferred stage of addition of polyglyceryl esters is the amalgamation/kneading of the mass at 65 °C that during the step C of the one pot process of present invention.

Polyglyceryl-4 laurate, which is made from polyglycerin-4 and pure lauric acid (C12, 99%), is procured from Galaxy surfactant Ltd. (It is a viscous liquid and viscosity at 45 °C 5500 cps, Sap value 140, Acid value is 5.1. HLB value 10, pH of 5 % dispersion in water is 6.0.) If original pH of this material is acidic then caustic lye (45 %) is used to adjust the pH of sigma kneaded material before it is taken for triple roll milling and plodding. In case of some commercially produced polyglyceryl -4 laurate (for ex TegoCare PL4 from Evonik) pH is on the acidic side then it can be adjusted to around 6.0 with sodium hydroxide and then can be used as mentioned in the experimental procedure in Example section. Alternatively, pH of sigma-mixed mass is adjusted (4.0 to 6.0 depending upon the intended benefit) before it is taken for refining and extruding. Typically, compositions for the feminine intimate hygiene application has the lowest pH of 3.5 to 4.0, the anti-acne compositions have pH of 4.5 to 5.5 and the pH of the compositions for general skin hygiene and scalp hygiene can be 5.5 to 6.0. Polyglyceryl-3 oleate is procured from Galaxy (Pale yellow colored liquid (Gardner 2.1), viscosity 1300 cps at room temp, Sap value 152, and pH of 5 % dispersion is 6.1. It is polyglyceryl-3 dioleate and HLB 4.4). Polyglyceryl esters of wide range seem to work as evidenced by Examples with polyglyceryl-3 oleate (low HLB of 4.4) and polyglyceryl-4 laurate (high HLB value of 9 to 10). The roll for polyglyceryl esters, with HLB values ranging from 4 to 10, in the Examples of the present invention is more of a binder, moisturizer, foam booster, irritancy reducer/emollient and not of an emulsifier with specific hydrophilic and hydrophobic balance. It is very likely that that polyglyceryl esters of higher HLB values can play the roll of a process aid, moisturizer and emollient as reported in EP 2468246.

Suitably the Polyglyceryl ester is present in an amount of at least 5 % by weight of the solid cleansing composition of present invention.

Suitably the polyglyceryl ester is present in an amount of at least 5 % by weight and up to 12 % by weight of the solid cleansing composition of present invention.

### Antimicrobials:

As used herein the antimicrobials, component e) of the solid cleansing composition of the present invention comprises one or more *N*-acyl glycines. The component e) is preferably added during the step C of the one pot process of present invention. The ingredients of component e) are *N*-capryloyl glycine (Formula I, CAS No 14246-53-8) and *N-*undecylenoyl glycine (Formula II, CAS No 54301-26-7). These *N*-acyl glycines can be used alone or in combination at concentration of 0.4 % to 4.0 % by weight of the total composition.

These two gentle antimicrobials are biodegradable. They are organic acids and are synthesized by combining vegetable fatty acids with glycine via a peptide linkage. The vegetable fatty acids are caprylic acid and undecylenic acid. Caprylic acid is derived from several vegetable oils whereas undecylenic acid is derived from castor oil. These are solid materials with melting point above 100°C (capryloyl glycine, M.P. 100-102°C and undecylenoyl glycine, M.P. 104 to 106°C) and they are incorporated at the step C of 'one-pot' sigma blending along with other thermo-insensitive materials and additives as described in the Examples 1 to 13. The solid cleansing compositions of the present invention employ either *N*-capryloyl glycine, or *N*-undecylenoyl glycine or in combination and they are used in powder form and the particle size distribution for both powders is as follows :100 % particles < 2000 microns, 90 % particles < 1200 and 50 % particles < 500 microns. These antimicrobial acyl glycines are added to the 'preformed' blend of hydrogenated oil, anionic surfactant (*O*-acyl isethionate) and glycerin at the second stage of the process where temperature of the amalgamated mass is 60 to 65 °C.

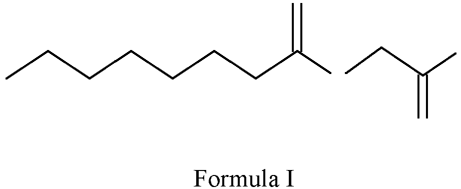

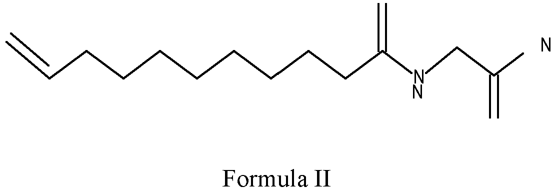

These gentle antimicrobials are well-established in personal care industry for their skin and scalp care benefits that stem from their broad range of antimicrobial activity. These are mostly deployed in a variety of `liquid' wash formulations (body wash/hair wash) and sometimes in 'leave-on' creams and lotions (anti-acne). These two *N*-acyl glycines exhibit anti-acne (against *Cutibacterium acnes)* and anti-dandruff (against *Malassezia restricta*) activities and are used in liquid format of both 'leave-on' and 'rinse-off applications. These two lipidated glycines, alone as well as when combined together, show remarkable activity against *Candida albicans* and *Candida glabrata* yeast *(vaginal candidiasis,* the yeast infection) and *Gardnerella vaginalis* (a Gram-variable bacterium that is a causative microbe for *bacterial vaginosis).* Interestingly, these two *N*-acyl glycines also exhibit a good activity against the microbiota of human axillary vault (typically against the prominent genera that reside in the axilla region, *Staphylococcus and Corynebacteria),* and are used in `malodour' control. These two *N*-acyl glycines, akin to skin fatty acids, are devoid of any structural feature, like halogen atom or a heterocycle, that can be a potential hazard to the environment and living systems. Hence, these gentle biodegradable `derma purifiers' are incorporated at minimum 0.4 % level by weight of total composition of the solid cleansers of the present invention for good hygiene of skin/hair/scalp. The N-acyl glycine alone or in combination can be used up to 4.0 % by weight of the total composition. The traditional environmentally toxic chlorinated antimicrobials for cleansing hygiene bars such as Chlorhexidne, Triclosan, Chloroxylenol, Climbazole, Triclocarban (banned in 2017) or other traditional anti-dandruff (antifungal) agents like Climbazole or Octopirox (monoethanol amine salt of piroctone), Zinc pyrithion (now banned in EU) etc. generate unsalvageable waste at every step of the multistep syntheses. In contrast to the facts (hazardous synthesis and toxicity) of traditional antimicrobials for solid cleansers, the lipidated glycines, that are part of solid compositions of the present invention, are manufactured by green chemistry without generating any unsalvageable/non-disposable waste as taught by IN patent 362888. Also, it is to be noted that *N*-acyl glycines are biodegradable and environmentally benign. These two lipidated glycines (Formula I and Formula II) are organic acids and by adding them in the second stage of processing when the mass of the first stage is being kneaded with limited amount of water in a sigma blender, helps to achieve the desired pH of the final solid cleanser. pH of the final solid cleanser can be brought down from 6.0 to 4.0 by incorporating these acyl glycines at an amount of about 0.5 % to 4.0 % of the total composition. The two *N*-acyl glycines can be used individually or in a combination.

Examples 1 to 9 deploy either *N*-capryloyl glycine or *N-*undecylenoyl glycine, however, Example 10 to13 use both of *N*-capryloyl glycine and *N*-undecylenoyl glycine. Higher % of capryloyl glycine (around 3.0 % of the total composition) results in lower pH that is intended for anti-acne or feminine intimate hygiene wash as exemplified in Example 3. Minimum inhibitory concentrations of capryloyl glycine (CG) and undecylenoyl glycine (UG) and their combination (CG-UG) in 1: 1 molar ratio using 1 % solution at pH 5.5 are given in Table 1. Overall, both *N*-acyl glycines exhibit good activity against *Malassezia* yeast as well as *Candida* yeast. The MIC numbers (less than 0.1 %) for the *N*-acyl glycines against *Gardnerella* bacterium are extremely good. In addition, the combination (1: 1 in molar ratio) of the two *N*-acyl glycines, shows synergy as reflected in MIC numbers (Table 1) against mold *Aspergillus niger, Gardnerella and Candida.*

**Table 1: Minimum Inhibitory Concentrations for A-acyl glycines**

| Minimum Inhibitory Concentration At pH 5.5 | **UG:CG (1:1)** | **CG** | **UG** |
|---|---|---|---|
| **Gram positive** | | | |
| *Staphylococcus hominis* MTCC 8980 | 0.2 | 0.3 | 0.2 |
| *Staphylococcus epidermidis* NCIM 2493 | 0.3 | 1 | 0.4 |
| *Staphylococcus aureus* ATCC 6538 | 0.5 | 1 | 0.6 |
| *Micrococcus luteus* NCIM 2103 | 0.3 | 1 | 0.5 |
| *Corynebacterium xerosis* ATCC 373 | 0.7 | 1. | 0.7 |
| *Propionibacterium acnes* MTCC 1951 | 0.6 | 1 | 1 |
| *Gardnerella vaginalis* ATCC 14018 | 0.05 | 0.09 | 0.06 |

| **Gram negative** | | | |
|---|---|---|---|
| *Escherichia coli* ATCC 8739 | 0.8 | 0.7 | 0.9 |
| *Pseudomonas aeruginosa* ATCC 15442 | 0.9 | 1 | 1 |
| *Burkholderia cepacia* ATCC 25416 | 0.9 | 1 | 1 |

| **Yeast** | | | |
|---|---|---|---|
| *Candida albicans* ATCC 10231 | 0.5 | 0.6 | 1 |
| *Candida glabrata* ATCC 66032 | 0.4 | 1 | 0.9 |
| *Malassezia furfur* ATCC 14521 | 0.5 | 0.5 | 0.5 |
| *Malassezia restricta* ATCC MYA 4611 | 0.4 | 0.4 | 0.4 |

| **Mold** | | | |
|---|---|---|---|
| *Aspergillus niger* ATCC 16404 | 0.7 | 1 | 1 |

Also, the *N*-acyl glycines individually show very low MIC numbers against all Gram-positive bacteria that are responsible for degrading sweat into mal-odorous molecules and the combination of two *N*-acyl glycines shows synergy against *Micrococcus, Staphylococcus and Corynebacterium* (Table 1). The preferred antimicrobials for the solid cleansers of the present invention are the combination of N-capryloyl glycine and *N-*undecylenoyl glycine in the molar ratio of 1:1.

The equimolar composition of *N*-capryloyl glycine and *N*-undecylenoyl glycine is incorporated in the syndet base (2 % of the total composition, Example 10) and compared with the syndet base that doesn't have the N-acyl glycines (the Blank composition). The bar cakes (2 % dispersion in water) are used for Time Kill study (five mins) against pathogenic microbes and against the odor causing (sweat degrading) microbes and results are tabulated in Table 2. The Blank is the one without *N*-acyl glycine. By virtue of being a surfactant, sodium cocoyl isethionate in the composition of the present invention exhibits some antimicrobial action. Control column in the Table 2 indicates the original concentration of microbes in terms of *cfu.* After five minutes of contact time, the original concentration of 10⁶ to 10⁷ cfu/ml of microbes, gets reduced by 1 to 2 logs for pathogens and 2 to 3 logs for Gram positives of axillary vault. What is significant is the difference between the colony count for 'the blank' and for the test bar with two *N*-acyl glycines (Table 2 and Table 3).

**Table 2: Time kill study (five mins) of syndet bars of Example 10 against pathogens**

| Pathogenic microbes | Control cfu/ml | Blank cfu/ml | Test cfu/ml |
|---|---|---|---|
| *Propionibacterium acnes* MTCC 1951 | 1×10⁷ | 1.2×10⁶ | 4.7×10⁵ |
| Log reduction | - | 0.92 | 1.26 |
| *Escherichia coli* ATCC 8739 | 2.9×10⁷ | 1×10⁷ | 1.7×10⁶ |
| Log reduction | - | 0.47 | 1.22 |
| *Candida albicans* ATCC 10231 | 2.9×10⁷ | 4×10⁵ | 3.5×10³ |
| Log reduction | - | 1.87 | 2.4 |

**Table 3: Time kill study (five mins) of syndet bars of Example 10 against odour generating axilla microbiota.**

| Axilla microbes | Control cfu/ml | Blank cfu/ml | Test cfu/ml |
|---|---|---|---|
| *Staphylococcus hominis* MTCC 8980 | 5.2×10⁶ | 4.9×10⁴ | 4.1×10² |
| Log reduction | - | 2.02 | 4.1 |
| *Corynebacterium xerosis* ATCC 373 | 7.3×10⁶ | 5.8×10⁶ | 5×10⁵ |
| Log reduction | - | 0.09 | 1.06 |
| *Staphylococcus epidermidis* NCIM 2493 | 1.3×10⁶ | 6.1×10⁴ | 3.1×10³ |
| Log reduction | - | 1.32 | 2.62 |
| *Micrococcus luteus* NCIM 2103 | 2.1×10⁶ | 3.4×10⁵ | 2.1×10³ |
| Log reduction | - | 0.79 | 2.98 |

Component f) of the grit free solid cleansing composition of present invention is water. Preferably the amount of water in the solid cleansing composition of present invention is less than 5% but more than 2% by weight of solid cleansing composition.

Preferably the water is present in an amount of less than 4 % by weight of the solid cleansing composition. Preferably the water is added during the step C of the one pot process of present invention. Preferably the amount of component f) is added in the solid composition such that it will not allow any hydrolysis of component c) of the solid cleansing composition. Furthermore, amount of water added in the solid cleansing composition of the present invention is for ease of kneading process. To aid or ease the kneading process the amount of water required is less than 5 % by weight of the cleansing composition.

### The Additives:

The additives referred to in the present invention are the anti-oxidants and the chelating agents. These are incorporated in the solid cleansers at step C of the one pot process of present invention. The anti-oxidant and the chelating agents are added along with *N*-acyl glycines and water. The preferred antioxidant for the present invention of solid composition is Vitamin E acetate that serves as skin benefit agent in addition to being useful for anti-oxidant properties.

Other tocopherol derivatives can be used in place of acetate derivative. Propyl gallate (CAS No 121-79-9) as anti-oxidant is used in Example 3.

The preferred chelating agents of the present invention are sodium gluconate (CAS No 527-07-1) or GLDA (tetrasodium glutamate diacetate, CAS No 51981-21-6). Most examples are with GLDA, however, Example 2 deploys sodium gluconate. Example 5 uses Etidronic acid, sodium salt (CAS no 7414-83-7) which is also popular chelating agent and an antioxidant. Any chelating agent can be used for the solid cleansers of the present invention however, GLDA, citric acid, and sodium gluconate are preferred for their eco-friendliness.

Preferably additive is selected from vitamin E acetate, propyl gallate, sodium gluconate, tetrasodium glutamate diacetate, sodium etidronate, citric acid and mixture thereof.

Preferably the additives are present in an amount 0 to 2 % by weight of the solid cleansing composition of present invention.

### The Actives:

The `actives' for the solid cleansing composition of the present invention are all types of benefit agents for hair and skin. The `heat-stable actives', just like 'additives' are incorporated in the composition during the one pot process step C of the present invention.

Suitably, the composition includes further optional actives, selected from but not limited to the hair care actives like vitamins, scalp-care agents, anti-dandruff agent, conditioners, UV-protectors, nourishing agents, moisturizing agents, shine/gloss agents, skin-care actives like moisturizers and conditioners, derma purifiers like antimicrobial, anti-acne agents, vitamins, nourishing agents, ceramides. Various Examples in the experimental section illustrate incorporation of some of the actives. For example, solid cleanser of Example 6 includes sodium stearoyl lactylate (emollient) and L-arginine (hair care active). Another example of the optional 'actives' is glyceryl monoundecylenate (Example 8, antimicrobial). The actives of Example 6 and 8 thermostable and hence can be incorporated when the solid mass in sigma is at 60-65°C. Another interesting multifuctional hare care active is methoxy cinnamidoproyl dimethyl behenyl chloride (CAS No 880645-41-0) which a multifunctional active (hair conditioner-cum-UV absorber). Being quaternary ammonium types of quaternized UV absorber, it shows some activity against Malassezia yeast and hence provides hygiene benefit in scalp-care (Example 17).

Other optional actives can be selected from the polymers of vegetable oils (CAS No 185323-46-0, copolymer of *Brassica campestris and Aleurites fordii*)*,* essential oils with unique properties like neem oil, tea tree oil, Lavender oil and cinnamon bark oil. Biosurfactants can be selected from sophorolipids (Example 9, made from oleic acid, glucose and the yeast, *Starmerella. bombicola*) or rhamnolipids or mannitol erythritol lipids. Other natural oils and skin benefit actives like almond oil, Baobab oil, or Jojoba oil can be included in the composition of solid cleanser. Vegetable extracts (like *Aloe vera)* can be part of solid cleansers and water-based extracts are added in the second step involving the kneading of the mass in a sigma blender. Heat sensitive actives like fragrances, oligopeptides, natural extracts are added when the temperature of solid mass in the blender is at ambient temperature or around 30 °C and are mixed for the adequate time to ensure the homogeneity of material.

Thus, the one pot process of preparing compositions of the present invention starts with softening of sodium *O*-acyl isethionate in preheated hydrogenated vegetable oils at 90 °C wherein the preferred form of anionic surfactant is powder of particle size less than 250 µm. The rest of the constituents (acyl glycines, polyglyceryl esters, water and chelating agent/antioxidants) are added to this pre-amalgamated solid at 65 °C and the blending operation is continued. The other heat-sensitive ingredients are added when the amalgamated mass is at room temperature and blending is continued. This mass is then further processed by refining (three roll mill) and plodding (a duplex plodder). The material produced in all the Examples is converted into bar cakes/tablets by conventional soap making equipment and are found to be of desired hardness and mush.

### Skin-scalp microbiota-friendly ingredients:

The ingredients of the solid cleansing compositions of this patent application are known for their mildness to the skin and hair. For example, the surfactant acyl isethionate is one of mildest anionic surfactant that gives creamy lather. Hydrogenated vegetable oils form the base of these composition that are the best natural emollients. The other two ingredients are glycerin and polyglyceryl esters that are the best moisturizer and emollients respectively. Polyglyceryl esters play multifunctional role of being binder (process aid) and foam booster in addition to imparting moisturization and providing emollience. All the four ingredients are gentle and friendly towards the natural balance of skin and scalp microbiota. The fifth ingredient is gentle antimicrobials that act on the hyperproliferating commensals of the microbiota and at the same time do not perturb the natural balance between skin microbiota and the host cells.

The invention is now described by way of non-limiting illustrative examples.

### EXAMPLES:

Sodium cocoyl isethionate (Galsoft SCI-85, anionic activity 85 %, particle size, < 250 µm), sodium cocoyl isethionate (Galsoft SCI-80, anionic activity 80 %, particle size, < 250 µm), sodium lauroyl isethionate (Galsoft SLI P (85 % active) in powder form (particle size less than 250 µm) and sodium lauroyl methyl taurate in powder form, Galsoft SLT-P (90 % active, particle size less than 250 µm) are supplied by Galaxy Surfactants Ltd. Mumbai, India. Hydrogenated soyabean oil, hydrogenated palm-stearin and hydrogenated sunflower oil are procured from AAK India Pvt Ltd.

Polyglyceryl-4 laurate (CAS No 75798-42-4) and Polyglyceryl -3 oleate (CAS No. 33940-98-6). are supplied by Galaxy Surfactants Ltd.

N-capryloyl glycine (CAS No 14246-53-8), *N*-undecylenoyl glycine (CAS No 54301-26-7) and glyceryl mono undecylenate (65684-27-7) are also supplied by Galaxy surfactants Ltd, Navi Mumbai, India. Capryloyl glycine and undecylenoyl glycine are in powder form and the particle size distribution for both powders is as follows :100 % particles < 2000 microns, 90 % particles < 1200 and 50 % particles < 500 microns. P-methoxycinnamoyl dimethyl behenyl ammonium chloride (GalHueShield HCS) is supplied by Galaxy surfactants Ltd. Sophorolipids (50% active) made from fermentation of oleic acid and glucose using wild strain of *Starmerella bombicola* ATCC2214, is supplied by Galaxy Surfactants India. It has 60% lactonic form and 40% acid form of sophorolipids.

Mush of the final bar cakes/tablets is determined by the protocol of `tablet immersion test `as described in IS 13498: 1997 of Bureau of Indian Standards. Mush is measured as loss of mass in gram per 50 cm² of the bar cake that is immersed in water for 2.5 hrs at 25 °C hours at ambient temperature.

Hardness of the bar cakes/tablets is measured by cone penetrometer as per ASTM D459.

Grittiness/sandiness (wet-bar feel of bar cakes/tablets) is evaluated as per the standard industry protocol described on page 424 of the chapter on `Soap Bar Performance Evaluation Methods' by Yury Yarovoy and Albert Post in the book titled `Soap Manufacturing Technology' Luis Spitz, second edition 2016, Academic press with AOCS press.

Moisture content is determined on Halogen moisture analyzer HX204 of Mettler Toledo.

### Example 1

To a stirred mixture of hydrogenated soyabean seed oil (360 g, 0.45 gmol), and glycerin (40 g, 0.43 gmol) at 90-95°C under nitrogen blanket in a jacketed sigma blender (with 'Z' type blades that rotate in opposite directions to each other), sodium cocoyl isethionate (powder 85 % active, particle size < 250 µm, 450 g, 1.10 gmol) is added slowly (about two hours) and stirring is continued for three hours. The mass in the sigma blender is cooled to 65°C under continuous kneading and to it, L-Glutamic acid, *N, N*-diacetic acid, tetrasodium salt, (2.0 g), Vitamin E acetate (5.0 g), polyglyceryl -3 oleate (90 g, 0.12 gmol), undecylenoyl glycine (powder, 10 g) and water (50.0 g) are added and the kneading operation is continued at 60-65 °C for 4 hours. The mass is then cooled to 30 to 35°C (995 g) while continuously kneading, refined using a triple roll mill and extruded using a duplex plodder (38-42 °C) to get the billets for stamping.

**Table 4 - Analysis of the bar cake of Example 1:**

| | |
|---|---|
| Moisture | 4.10 % |
| pH (5 % dispersion in water) | 5.60 |
| Active matter (as sodium cocoyl isethionate, MW 347) | 37.41% |
| Hardness | 8 to 9 |
| Grit | Absent |
| Mush | 5.4 g /50 cm² |

### Example 2

To a stirred mixture of hydrogenated soyabean seed oil (360 g, 0.45 gmol), and glycerin (40 g, 0.43 gmol) at 70-75 °C under nitrogen blanket in a jacketed sigma blender (with 'Z' type blades that rotate in opposite directions to each other), sodium cocoyl isethionate (powder, 80 % active with particle size < 250 µm, 450 g, (1.10 gmol) is added slowly (about two hours) and stirring is continued for three hours. The blended mass is then cooled to 60 °C and to it, sodium gluconate (2.0 g), Vitamin E acetate, (5.0 g), polyglyceryl -4 laurate (90 g, 0.18 gmol), undecylenoyl glycine, (powder, 10 g) and water (50.0 g) are added, and the kneading is continued at same temperature for 4 hours. The mass is further cooled to 30 to 35 °C while continuously kneading (992 g), refined using a triple roll mill and extruded using a duplex plodder (38-42 °C) to get the billets for stamping.

**Table 5 - Analysis of the bar cake of Example 2:**

| | |
|---|---|
| Moisture | 3.90% |
| pH (5 % dispersion in water) | 5.8 |
| Active matter as sodium cocoyl isethionate, (MW 347) | 37.92 % |
| Hardness | 8 |
| Grit | Absent |
| Mush | 5.2 g /50 cm² |

### Example 3

To a stirred mixture of hydrogenated soyabean seed oil (360 g, 0.44 gmol), and glycerin (40 g, 0.43 gmol) in a sigma blender (with 'Z' type blades that rotate in opposite directions to each other) at 90-95 °C under nitrogen blanket, sodium cocoyl isethionate (powder, 85 % active with particle size < 250 µm, 450 g, 1.10 gmol) is added slowly (about two hour) and stirring is continued for three hours. The mass is cooled to 65 °C and to it, *Etidronic acid* (1-hydroxyethane 1,1-diphosphonic acid disodium salt) (2.0 g), propyl gallate (5.0 g), polyglyceryl -4 laurate (90 g, 0.18 gmol), capryloyl glycine, (powder, 30.0 g) and water (50.0 g) are added and kneading of the mass is continued for additional four hours at the same temperature. The mass is further cooled to 30 to 35 °C while continuously kneading (1012 g), refined using a triple roll mill and extruded using a duplex plodder (38-42°C) to get the final billets for stamping.

**Table 6 - Analysis of the bar cake of Example 3:**

| | |
|---|---|
| Moisture | 4.15 % |
| pH (5 % dispersion in water) | 4.2 |
| Anionic active matter as sodium cocoyl isethionate content (MW347) | 37.86 % |
| Hardness | 10 |
| Grit | Absent |
| Mush | 5.32 g /50 cm² |

### Example 4

To a stirred mixture of hydrogenated soyabean seed oil (360 g, 0.44 gmol), polyglyceryl -4 laurate (90 g, 0.18 gmol), and glycerin (40.g, 0.43 gmol) at 90-95°C under nitrogen blanket in a jacketed sigma blender (with 'Z' type blades that rotate in opposite directions to each other), sodium cocoyl isethionate (powder, 85 % active with particle size < 250 µm, 450 g, 1.10 gmol) is added slowly over two hours and mixing is continued for three hours. The mass in the sigma blender is cooled to 65 °C under continuous mixing.

To this kneaded mass, L-Glutamic acid, *N, N*-diacetic acid, tetrasodium salt, (2.0 g), Vitamin E acetate (5.0 g), capryloyl glycine, (powder, 10 g) and water (48.0 g) are added and continued to blend at 65 °C for four hours. The mass is cooled with continued mixing/kneading to room temperature (999.0 g), refined using a triple roll mill and extruded using a duplex plodder (38-42°C) to get the billets for stamping.

**Table 7 - Analysis of the bar cake of Example 4:**

| | |
|---|---|
| Moisture | 4.30% |
| pH (5% dispersion in water) | 5.4 |
| Active matter as sodium cocoyl isethionate, (MW 347) | 37.63 % |
| Hardness | 9 |
| Grit | Absent |
| Mush | 5.70 g /50 cm² |

### Example 5

To a stirred mixture of hydrogenated soyabean seed oil (360 g, 0.44 gmol), and glycerin (40 g, 0.43 gmol) at 90-95°C under nitrogen blanket in a jacketed sigma blender (with 'Z' type blades that rotate in opposite directions to each other), sodium lauroyl isethionate (powder, 85 % active with particle size < 250 µm, (450 g, 1.10 gmol) is added slowly over a period of two hours and stirring is continued for additional three hours. The blended mass is cooled to 65°C and to it, 1-hydroxyethane 1,1-diphosphonic acid disodium salt (Etidronic acid, 3.0 g), tocopherol acetate (10.0 g), polyglyceryl -4 laurate (90 g, 0.18 gmol) capryloyl glycine (20 g, powder) and water (50 g) are added, and mixing is continued at 60-65 °C for 4 hours. The kneaded mass is further cooled down to 30°C (1009.0 g), refined using a triple roll mill and extruded using a duplex plodder (38-42°C) to get the billets for stamping.

**Table 8 - Analysis of the bar cake of Example 5:**

| | |
|---|---|
| Moisture | 3.90% |
| pH (5 % dispersion in water) | 4.3 |
| Active matter as sodium lauroyl isethionate, (MW327) | 36.73 % |
| Hardness | 10 |
| Grit | Absent |
| Mush | 5.73 g /50 cm² |

### Example 6

To a stirred mixture of hydrogenated sunflower seed oil (360g, 0.44 gmol), and glycerin (40 g, 0.43 gmol) at 90-95 °C under nitrogen blanket in a jacketed sigma blender (with 'Z' type blades that rotate in opposite directions to each other), sodium lauroyl isethionate (powder, 85 % active particle size < 250 µm, 450g, 1.10 gmol) is added slowly and stirring is continued for three hours. The blended mass is cooled to 65°C under mixing. To it, L-Glutamic acid, *N, N*-diacetic acid, tetrasodium salt, (2.0 g), polyglyceryl -4 laurate (90 g 0.18 gmol), capryloyl glycine (powder, 8.0 g), vitamin E acetate (5.0 g) and sodium stearoyl lactylate, (5.0 g), L-arginine (5.0 g) and water (48.0 g) are added and blending is continued at 60-65°C for 4 hour. The mass is further cooled to 30°C (1002.0 g), refined using a triple roll mill and extruded using a duplex plodder (38-42°C) to get the billets for stamping.

**Table 9 - Analysis of the bar cake of Example 6:**

| | |
|---|---|
| Moisture | 4.35 % |
| pH (5 % dispersion in water) | 5.80 |
| Active matter as sodium lauroyl isethionate, (MW 347) | 37.62 % |
| Hardness | 9.5 |
| Grit | Absent |
| Mush | 5.80 g /50 cm² |

### Example 7

To a stirred mixture of hydrogenated soyabean seed oil (350 g, 0.44 gmol), and glycerin (40 g, 0.43 gmol) at 90-95°C under nitrogen blanket in a jacketed sigma blender (with 'Z' type blades that rotate in opposite directions to each other), sodium lauroyl isethionate (powder, particle size < 250 µm, 350 g, 0.85 gmol), followed by sodium lauroyl N-methyl taurate (powder with particle size < 250 µm, 90 % active (100 g, 0.26 gmol)), are added slowly (two hour) and stirring is continued for 3 hours. The blended mass is cooled to 60-65°C. To this kneaded mass, L-Glutamic acid, *N, N*-diacetic acid, tetrasodium salt (2.0 g), tocopherol acetate (5.0 g), polyglyceryl -4 laurate (90 g, 0.18 gmol), capryloyl glycine, (powder, 10.0 g) and water (50 g) are added and kneading/blending is continued at 60-65°C for 4 hour. The mass is cooled to 30°C (985 g), refined using a triple roll mill and extruded using a duplex plodder (38-42 °C) to get the final billets for stamping.

**Table 10 - Analysis of the bar cake of Example 7:**

| | |
|---|---|
| Moisture | 4.35 % |
| pH (5 % dispersion in water) | 5.3 |
| Active matter (on average MW of 345 (based on the molar ratio of sodium cocoyl isethionate and sodium lauroyl *N*-methyl taurate) | 38.12 % |
| Hardness | 10 |
| Grit | Absent |
| Mush | 6.83 g /50 cm² |

### Example 8

To a stirred mixture of hydrogenated Palm-Stearin oil (360 g, 0.44 gmol), and glycerin (40 g, 0.43 gmol) at 90-95 °C under nitrogen blanket in a jacketed sigma blender (with 'Z' type blades that rotate in opposite directions to each other), sodium cocoyl isethionate (powder, 85 % active with particle size < 250 µm, (450 g, 1.10 gmol) is added slowly (about two hour) and stirring is continued for three hours. The blended mass is cooled to 65 °C.

To this kneaded mass, 1-hydroxyethane 1,1-diphosphonic acid disodium salt (chelating agent, *Etidronic acid,* 2.0 g), propyl gallate (anti-oxidant, 5.0 g), polyglyceryl -4 laurate (90 g, 0.18 gmol), capryloyl glycine (powder, 6.0 g), glyceryl undecylenate (5.0 g) and water (50.0 g) are added and mixing is continued at 60-65 °C for 4 hour. The mass is further cooled to 30 to 35 °C while continuously kneading (990 g), refined using a triple roll mill and extruded using a duplex plodder (38-42 °C) to get the final billets for stamping.

**Table 11 - Analysis of the bar cake of Example 8:**

| | |
|---|---|
| Moisture | 4.58 % |
| pH (5 % dispersion in water) | 5.6 |
| Active matter as sodium cocoyl isethionate content (MW347) | 37.61 % |
| Hardness | 9.5 |
| Grit | Absent |
| Mush | 3.50 g /50 cm² |

### Example 9

To a stirred mixture of hydrogenated Palm-Stearin oil (360 g, 0.44 gmol), and glycerin (40 g, 0.43 gmol) at 90-95 °C under nitrogen blanket in a jacketed sigma blender (with 'Z' type blades that rotate in opposite directions to each other), sodium cocoyl isethionate (powder, 85 % active with particle size < 250 µm, (450 g, 1.10 gmol) is added slowly (two hours) and stirring is continued for three hours. The blended mass is cooled to 65 °C.

To the kneaded mass, L-Glutamic acid, *N, N*-diacetic acid, tetrasodium salt (2.0 g), tocopherol acetate (5.0 g), polyglyceryl -4 laurate (90 g, 0.18 gmol), capryloyl glycine (7.0 g), sophorolipids (20.0 g, 50 % active) and water (40 g) are added and mixing is continued at 60-65 °C for 4 hour. The mass is further cooled to 30 to 35 °C while continuously kneading (996.0 g), refined using a triple roll mill and extruded using a duplex plodder (38-42 °C) to get the billets for stamping.

**Table 12 - Analysis of the bar cake of Example 9:**

| | |
|---|---|
| Moisture | 4.58 % |
| pH (5 % dispersion in water) | 5.8 |
| Active matter as sodium cocoyl isethionate content (MW347) | 37.61 % |
| Hardness | 9.5 |
| Grit | Absent |
| Mush | 3.50 g /50 cm² |

### Example 10

To a stirred mixture of hydrogenated palm-stearin oil (360 g, 0.44 gmol), and glycerin (40 g, 0.43 gmol) at 90-95 °C under nitrogen blanket in a jacketed sigma blender (with 'Z' type blades that rotate in opposite directions to each other), sodium cocoyl isethionate (powder, 85 % active with particle size < 250 µm, (450 g, 1.10 gmol) is added slowly (about two hour) and stirring is continued for three hours. The blended mass is cooled to 65 °C.

To this kneaded mass, L-Glutamic acid, *N, N*-diacetic acid, tetrasodium salt (2.0 g), tocopherol acetate (5.0 g), polyglyceryl -4 laurate (90 g, 0.18 gmol), water (50 g), capryloyl glycine (powder, 10.0 g), and undecylenoyl glycine (powder, 10.0 g) are added and mixing is continued at 60-65 °C for 4 hours. The mass is further cooled to 30 to 35 °C while continuously kneading (1008.0 g), refined using a triple roll mill and extruded using a duplex plodder (38-42 °C) to get the billets for stamping.

**Table 13 - Analysis of the bar cake of Example 10:**

| | |
|---|---|
| Moisture | 4.00 % |
| pH (5 % dispersion in water) | 4.3 |
| Active matter as sodium cocoyl isethionate content (MW347) | 38.0% |
| Hardness | 9.0 |
| Grit | Absent |
| Mush | 3.50 g /50 cm² |

The above process/experiment is repeated, and the solid cleansing composition is made without *N*-acyl glycines. This composition is referred to as 'the Blank'. The solid cleansing compositions of Example 10 and the corresponding 'the Blank' are used for `Time kill' study by preparing 2.0 % w/w dispersion in distilled water using of the final stamped bar cake. The organisms selected are the pathogens (Table 2, discussion section) and residents of human axillary vault (Table 3, discussion section) that degrade sweat into odorous molecules. The relative drop in cfu as seen in the log reduction of microbial count using composition of Example 10 (TEST) versus 'the blank' after five minutes of contact are tabulated in Table 2 and Table 3.

### Example 11

To a stirred mixture of hydrogenated palm-stearin oil (360 g, 0.44 gmol), and glycerin (40 g, 0.43 gmol) at 90-95°C under nitrogen blanket in a jacketed sigma blender (with 'Z' type blades that rotate in opposite directions to each other), sodium cocoyl isethionate (powder, 85% active with particle size < 250 µm, (450 g, 1.10 gmol) is added slowly (two hours) and stirring is continued for three hours. The blended mass is cooled to 65 °C.

To this sigma-kneaded mass, L-Glutamic acid, *N, N*-diacetic acid, tetrasodium salt (2.0 g), tocopherol acetate (5.0 g), polyglyceryl -4 laurate (90 g, 0.18 gmol), water (48 g), capryloyl glycine (powder, 20.0 g), and undecylenoyl glycine (powder, 20.0 g) are added and mixing is continued at 60-65 °C for 4 hours. The mass is further cooled to 30 to 35 °C while continuously kneading (1005.0 g), refined using a triple roll mill and extruded using a duplex plodder (38-42°C) to get the billets for stamping.

**Table 14 - Analysis of the bar cake of Example 11:**

| | |
|---|---|
| Moisture | 4.00 % |
| pH (5 % dispersion in water) | 3.9 |
| Active matter as sodium cocoyl isethionate content (MW347) | 38.4 % |
| Hardness | 8.0 |
| Grit | Absent |
| Mush | 4.0 g /50 cm² |

### Example 12

To a stirred mixture of hydrogenated soya bean oil (360 g, 0.44 gmol), and glycerin (40 g, 0.43 gmol) at 90-95°C under nitrogen blanket in a jacketed sigma blender (with 'Z' type blades that rotate in opposite directions to each other), sodium cocoyl isethionate (powder, 85 % active with particle size < 250 µm, (450 g, 1.10 gmol) is added slowly (about two hour) and stirring is continued for three hours. The blended mass is then cooled to 60-65°C.

To this blended mass, L-Glutamic acid, *N, N*-diacetic acid, tetrasodium salt (2.0 g), tocopherol acetate (5.0 g), polyglyceryl -3 oleate (90 g, 0.12 gmol), water (50 g), capryloyl glycine (powder, 10.0 g), and undecylenoyl glycine (powder, 10.0 g) are added and mixing is continued at 60-65 °C for 4 hour. The mass is further cooled to 30 to 35°C while continuously kneading, refined using a triple roll mill and extruded (1005 g) using a duplex plodder (38-42°C) to get the billets for stamping.

**Table 15 - Analysis of the bar cake of Example 12:**

| | |
|---|---|
| Moisture | 4.8 % |
| pH (5 % dispersion in water) | 4.8 |
| Active matter as sodium cocoyl isethionate content (MW347) | 38.0 % |
| Hardness | 8 |
| Grit | Absent |
| Mush | 7.5 g /50 cm² |

### Example 13

To a stirred mixture of hydrogenated palm-stearin oil (360 g, 0.44 gmol), and glycerin (40 g, 0.43 gmol) at 90-95°C under nitrogen blanket in a jacketed sigma blender (with 'Z' type blades that rotate in opposite directions to each other), sodium cocoyl isethionate (powder, 85% active with particle size < 250 µm, (450 g, 1.10 gmol) is added slowly (about one hour) and stirring is continued for three hours. The blended mass is cooled to 60-65°C.

To this blended mass at 65 °C, L-Glutamic acid, *N, N*-diacetic acid, tetrasodium salt (2.0 g), tocopherol acetate (5.0 g), polyglyceryl -3 oleate (90 g, 0.122 gmol), water (48 g), capryloyl glycine (powder, 20.0 g), and undecylenoyl glycine (powder, 20.0 g) are added and mixing is continued at 60-65 °C for 4 hours. The mass is further cooled to 30 to 35°C while continuously kneading, refined using a triple roll mill and extruded (1012 g) using a duplex plodder (38-42 °C) to get the final billets for stamping.

**Table 16 - Analysis of the bar cake of Example 13:**

| | |
|---|---|
| Moisture | 4.3 % |
| pH (5 % dispersion in water) | 3.9 |
| Active matter as sodium cocoyl isethionate content (MW347) | 38.4 % |
| Hardness | 7 |
| Grit | Absent |
| Mush | 4.0 g /50 cm² |

### Example 14

To a stirred mixture of hydrogenated palm-stearin oil (438 g, 0.53 gmol), and polyglycerin-4 (48 g, 0.15 gmol) at 90-95 °C under nitrogen blanket in a jacketed sigma blender (with 'Z' type blades that rotate in opposite directions to each other), sodium cocoyl isethionate (powder, 85 % active with particle size < 250 µm, (540 g, 1.32 gmol) is added slowly (about one hour) and stirring is continued for three hours. The blended mass is cooled to 60-65 °C.

To this blended mass at 65 °C, L-Glutamic acid, *N, N*-diacetic acid, tetrasodium salt (2.0 g), tocopherol acetate (2.5 g), polyglyceryl -4 laurate (108 g, 0.21 gmol), water (55 g), and capryloyl glycine (powder, 8.0 g) are added and mixing is continued at 60-65°C for 4 hours. The mass is further cooled to 30 to 35°C while continuously kneading, refined using a triple roll mill and extruded (1195 g) using a duplex plodder (38-42°C) to get the final billets for stamping.

**Table 17 - Analysis of the bar cake of Example 14:**

| | |
|---|---|
| Moisture | 4.1 % |
| pH (5 % dispersion in water) | 5.7 |
| Active matter as sodium cocoyl isethionate content (MW347) | 38.73 % |
| Hardness | 8 |
| Grit | Absent |
| Mush | 4.5 g /50 cm² |

### Example 15

To a stirred mixture of hydrogenated palm-stearin oil (438 g, 0. 53gmol), and polyglycerin-3 (48 g, 0.19 gmol) at 90-95 °C under nitrogen blanket in a jacketed sigma blender (with 'Z' type blades that rotate in opposite directions to each other), sodium cocoyl isethionate (powder, 85 % active with particle size < 250 µm, (540 g, 1.32 gmol) is added slowly (about one hour) and stirring is continued for three hours. The blended mass is cooled to 60-65 °C.

To this blended mass at 65 °C, L-Glutamic acid, *N, N*-diacetic acid, tetrasodium salt (2.0 g), tocopherol acetate (2.5 g), polyglyceryl -4 laurate (108 g, 0.21 gmol), water (55 g), and capryloyl glycine (powder, 8.0 g) are added and mixing is continued at 60-65 °C for 4 hours. The mass is further cooled to 30 to 35 °C while continuously kneading, refined using a triple roll mill and extruded (1197 g) using a duplex plodder (38-42 °C) to get the final billets for stamping.

**Table 18 - Analysis of the bar cake of Example 15:**

| | |
|---|---|
| Moisture | 4.34 % |
| pH (5 % dispersion in water) | 5.6 |
| Active matter as sodium cocoyl isethionate content (MW347) | 38.06 % |
| Hardness | 8 |
| Grit | Absent |
| Mush | 5.1 g /50 cm² |

### Example 16

To a stirred mixture of hydrogenated palm-stearin oil (438 g, 0.53 gmol), and 1,3-propane diol (65 g, 0.85 gmol) at 90-95 °C under nitrogen blanket in a jacketed sigma blender (with 'Z' type blades that rotate in opposite directions to each other), sodium cocoyl isethionate (powder, 85 % active with particle size < 250 µm, (540 g, 1.32 gmol) is added slowly (about one hour) and stirring is continued for three hours. The blended mass is cooled to 60-65 °C.

To this blended mass at 65 °C, L-Glutamic acid, *N, N*-diacetic acid, tetrasodium salt (2.0 g), tocopherol acetate (2.5 g), polyglyceryl -4 laurate (108 g, 0.21gmol), water (55 g), and capryloyl glycine (powder, 8.0 g) are added and mixing is continued at 60-65 °C for 4 hours. The mass is further cooled to 30 to 35 °C while continuously kneading, refined using a triple roll mill and extruded (1201 g) using a duplex plodder (38-42 °C) to get the final billets for stamping.

**Table 19 - Analysis of the bar cake of Example 16:**

| | |
|---|---|
| Moisture | 4.8 % |
| pH (5 % dispersion in water) | 5.7 |
| Active matter as sodium cocoyl isethionate content (MW347) | 37.0 % |
| Hardness | 9 |
| Grit | Absent |
| Mush | 4.6 g /50 cm² |

### Example 17

To a stirred mixture of hydrogenated palm-stearin oil (438 g, 0.53 gmol), and polyglycerin-4 (48 g, 0.15 gmol) at 90-95°C under nitrogen blanket in a jacketed sigma blender (with 'Z' type blades that rotate in opposite directions to each other), sodium cocoyl isethionate (powder, 85 % active with particle size < 250 µm, (540 g, 1.32 gmol) is added slowly (about one hour) and stirring is continued for three hours. The blended mass is cooled to 60-65 °C.

To this blended mass at 65 °C, L-Glutamic acid, *N, N*-diacetic acid, tetrasodium salt (2.5 g), tocopherol acetate (2.5 g), polyglyceryl -4 laurate (108 g, 0.21 gmol), water (55 g), p-methoxy cinnamidopropyl dimethyl behenyl ammonium chloride (12.0 g) and capryloyl glycine (powder, 8.0 g) are added and mixing is continued at 60-65 °C for 4 hours. The mass is further cooled to 30 to 35 °C while continuously kneading, refined using a triple roll mill and extruded (1191 g) using a duplex plodder (38-42 °C) to get the final billets for stamping.

**Table 20 - Analysis of the bar cake of Example 17:**

| | |
|---|---|
| Moisture | 4.47 % |
| pH (5 % dispersion in water) | 5.7 |
| Active matter as sodium cocoyl isethionate content (MW347) | 38.00 % |
| Hardness | 8 |
| Grit | Absent |
| Mush | 6.0 g /50 cm² |

### Advantages of the present Invention

1) The present invention provides a one pot process making 'grit-free', 'sulphate-free', 'soap-free' solid cleansing bar using sodium O-acyl isethionates without having to resort to any emulsification with water.
2) The one pot process of the present invention avoids excess use of water and temperature as reported in the prior art and hence obviates the hydrolysis of ester type of surfactant, namely, sodium O-acyl isethionate. In the one pot process of the present invention, there is no involvement of water when the surfactants in microfine powder form are mixed with hydrogenated triglyceride oils.
3) The ingredients of acidic nature can be used without any concern for acid catalyzed hydrolysis of ester type surfactants during the process of amalgamation. This allows creation of compositions with very low acidic pH as in anti-acne cleanser or feminine intimate hygiene wash where the desired pH is in the range of 3.5 to 4.5.
4) The compositions of the present invention exploit the synergy between two gentle, skin microbiota-friendly, non-toxic, biodegradable antimicrobials, capryloyl glycine and undecylenoyl glycine for a variety of personal care benefits that include, anti-acne, anti-dandruff, anti-malodor, feminine intimate hygiene and in general, body cleansers with skin and scalp hygiene.
5) The one pot process of the present application is amenable to create solid cleansers with incorporation of high percentage of vegetable oil/s thus resulting in high emollience in skin care and high conditioning in case of hair care.
6) The solid cleansers obtained from the current process have the right balance of hardness and mush while delivering good performance on gentle cleansing with copious lather.
7) The one pot process of present invention requires lesser energy consumption as there is no requirement of addition of excess water and subsequently evaporating/reducing the water content as water being used in the composition of present invention is just to facilitate kneading and extrusion.

## Claims

1. A grit-free solid cleansing composition comprising:
a) at least 30 %, by weight of the total composition, one or more hydrogenated vegetable oil;
b) at least 4% by weight of the total composition, a humectant;
c) at least 30 %, by weight of the total composition, a salt of O-acyl isethionate;
d) at least 5 %, by weight of the total composition, a polyglyceryl ester of fatty acid;
e) at least 0.4 % by weight of the total composition, one or more N-acyl glycine; and
f) less than 5 % by weight of the total composition, water,
wherein, pH of the grit-free solid cleansing composition at 5 % dispersion in water, is between 3.5 to 6.0,
wherein melting point of the hydrogenated vegetable oil is at least 45 °C; and
wherein the salt of O-acyl isethionate is in powder form with particle size less than 250 µm.

2. A grit-free solid cleansing composition comprising:
a) 30 % to 40 % by weight of the total composition, one or more hydrogenated vegetable oil;
b) 4% to 10 % by weight of the total composition, a humectant;
c) 30 % to 45 % by weight of the total composition, a salt of O-acyl isethionate;
d) 5 % to 12 % by weight of the total composition, a polyglyceryl ester of fatty acid;
e) 0.4 % to 4 % by weight of the total composition, one or more N-acyl glycine; and
f) less than 5 % by weight of the total composition, water,
wherein, pH of the grit-free solid cleansing composition at 5 % dispersion in water, is between 3.5 to 6.0,
wherein melting point of the hydrogenated vegetable oil is at least 45 °C; and
wherein the salt of O-acyl isethionate is in powder form with particle size less than 250 µm.

3. The grit-free solid cleansing composition as claimed in claims 1 or 2, wherein the salt of O-acyl isethionate is selected from sodium cocoyl isethionate, sodium lauroyl isethionate, potassium cocoyl isethionate, potassium lauroyl isethionate or mixture thereof.

4. The grit-free solid cleansing composition as claimed in one or more of claims 1 to 3, wherein the one or more hydrogenated vegetable oil is selected from hydrogenated soyabean oil, hydrogenated sunflower oil, hydrogenated palm-stearin, hydrogenated cotton seed oil or mixture thereof.

5. The grit-free solid cleansing composition as claimed in one or more of claims 1 to 4, wherein the polyglyceryl ester of fatty acid is selected from polyglyceryl-4 laurate, polyglyceryl-3 oleate or mixture thereof.

6. The grit-free solid cleansing composition as claimed in one or more of claims 1 to 5, wherein the humectant is selected from Glycerin, sorbitol, polyglycerin, 1,3-propane diol or mixture thereof.

7. The grit-free solid cleansing composition as claimed in one or more of claims 1 to 6, wherein the N-acyl glycine is selected from N-capryloyl glycine, N-undecylenoyl glycine or mixture thereof.

8. The grit-free solid cleansing composition as claimed in one or more claims 1 to 7, wherein the composition further comprises an additive.

9. The grit-free solid cleansing composition as claimed in claim 8, wherein the additive is selected from vitamin E acetate, propyl gallate, sodium gluconate, tetrasodium glutamate diacetate, sodium etidronate, citric acid and mixture thereof.

10. A one pot process for preparing grit-free solid cleansing composition comprising:
a) at least 30 %, by weight of the total composition, one or more hydrogenated vegetable oil;
b) at least 4% by weight of the total composition, a humectant;
c) at least 30 %, by weight of the total composition, a salt of O-acyl isethionate;
d) at least 5 %, by weight of the total composition, a polyglyceryl ester of fatty acid;
e) at least 0.4 % by weight of the total composition, one or more N-acyl glycine; and
f) less than 5 % by weight of the total composition, water,
wherein, pH of the solid composition at 5 % dispersion in water, is between 3.5 to 6.0, wherein melting point of the hydrogenated vegetable oil is at least 45 °C;
wherein the salt of O-acyl isethionate is in powder form with particle size less than 250 µm; and
wherein the process comprises the steps of:
A. mixing component a) and b) at temperature between 90 to 95 °C, to afford a homogenous mass;
B. adding component c) to the homogenous mass of step A to obtain a reaction mass;
C. cooling the reaction mass of step B and adding components d) to f) followed by kneading to afford an amalgamated mass;
D. processing the amalgamated mass of step C into a billets; and.
E. processing the billets obtained in step D into the solid cleansing composition.

11. The one pot process as claimed in claim 10, wherein the salt of O-acyl isethionate is selected from sodium cocoyl isethionate, sodium lauroyl isethionate, potassium cocoyl isethionate, potassium lauroyl isethionate or mixture thereof.

12. The one pot process as claimed in claim 10 or 11, wherein the one or more hydrogenated vegetable oil is selected from hydrogenated soyabean oil, hydrogenated sunflower oil, hydrogenated palm-stearin, hydrogenated cotton seed oil or mixture thereof.

13. The one pot process as claimed in one or more of claims 10 to 12, wherein
a) the polyglyceryl ester of fatty acid is selected from polyglyceryl-4 laurate, polyglyceryl-3 oleate or mixture thereof;
and/or
b) the humectant is selected from Glycerin, sorbitol, polyglycerin, 1,3-propane diol or mixture thereof.
and/or
c) the N-acyl glycine is selected from N-capryloyl glycine, N-undecylenoyl glycine or mixture thereof.

14. The one pot process as claimed in one or more of claims 10 to 13, wherein the solid cleansing composition further comprises an active and additive, wherein the active and additive are added to the composition at step C of the one pot process.

15. The one pot process as claimed in one or more of claims 10 to 14, wherein the reaction mass during step C is cooled to temperature between 60 to 65 °C.
